# EUROPÄISCHE PATENTSCHRIFT

(11) **EP 2 299 163 B1**
(45) Veröffentlichungstag und Bekanntmachung des Hinweises auf die Patenterteilung: **10.07.2019**
(21) Anmeldenummer: 10009349.1
(22) Anmeldetag: 08.09.2010
(51) Int. Cl.: F21S 8/00, F21V 7/04, F21Y 115/10, F21Y 103/33, F21W 131/205

(54) **Operationsleuchte**
Operating light
Lampe chirurgicale

(30) Priorität: 21.09.2009 DE 102009042338
(43) Veröffentlichungstag der Anmeldung: 23.03.2011
(73) Patentinhaber: Berchtold GmbH & Co. KG, 78532 Tuttlingen (DE)
(72) Erfinder: Jacobi, Leif, 78532 Tuttlingen (DE)
(74) Vertreter: Manitz Finsterwald Patent- und Rechtsanwaltspartnerschaft mbB

(56) Entgegenhaltungen:
- EP-A1- 2 023 035
- EP-A2- 1 152 187
- DE-A1-102005 036 275
- US-A- 4 745 526
- US-A1- 2007 217 193

## Beschreibung

Die vorliegende Erfindung betrifft eine Operationsleuchte nach dem Oberbegriff des Anspruchs 1 (vgl. US 4 745 526 A). Eine weitere Operationsleuchte mit einem ringförmigen Reflektor und mit LEDs ist aus der DE 10 2005 036 275 A1 bekannt.

Bei Operationsleuchten, die das auf ein Operationsfeld zu richtende Licht mit Hilfe von LEDs erzeugen, ist einerseits die Wärmeentwicklung der LEDs, andererseits die gleichmäßige Ausleuchtung des Operationsfelds ohne Farbschatten sowie ohne Schlagschattenbildung problematisch.

Es ist die Aufgabe der vorliegenden Erfindung, eine Operationsleuchte nach dem Oberbegriff des Anspruchs 1 so weiterzubilden, dass bei guter Wärmeableitung auch in unterschiedlichen Arbeitsentfernungen von der Operationsleuchte ein gleichmäßig ausgeleuchtetes Leuchtfeld erzeugt werden kann, das so gut wie keine Schattenbildung aufweist, wenn ein Teil des auf das Operationsfeld auftreffenden Lichtes durch den Operateur abgeschattet wird.

Die Lösung dieser Aufgabe erfolgt durch die Merkmale des Anspruchs 1.

Der Hauptreflektor ist in einzelne Segmente bzw. Zonen oder auch Facetten unterteilt, wobei der Übergang zwischen benachbarten Segmenten (im mathematischen Sinn) unstetig oder auch stetig verlaufen kann. Erfindungsgemäß sind dabei einzelne Zonen (Facetten) des Hauptreflektors in mindestens zwei Gruppen unterteilt, wobei jede Gruppe von Reflektorzonen das einfallende Licht auf eine andere Fokussierebene abbildet. Hierdurch sind verschiedene Reflektorzonen für unterschiedliche Arbeitsentfernungen geschaffen und es wird gegenüber herkömmlichen Operationsleuchten eine deutlich verbesserte Tiefenausleuchtung erreicht.

Vorteilhafte Ausführungsformen der Erfindung sind in der Beschreibung, den Zeichnungen sowie den Unteransprüchen beschrieben.

Nach einer vorteilhaften Ausführungsform können die LEDs so angeordnet sein, dass deren Licht über zumindest einen Einkoppelreflektor in den Hauptreflektor eingekoppelt wird.

Der Einkoppelreflektor kann nach einer weiteren vorteilhaften Ausführungsform ein Ringreflektor sein, der im Bereich des Außenrandes des Hauptreflektors angeordnet ist oder aber der einstückig mit dem Hauptreflektor ausgebildet ist. Bei der zuletzt genannten Ausführungform kann der Hauptreflektor dann mit Öffnungen versehen sein, durch die das Licht der LEDs von der Rückseite des Hauptreflektors aus auf den Einkoppelreflektor und von dort auf den Hauptreflektor gestrahlt wird.

Um das Auftreten von Farbschatten zu minimieren, können entlang des Außenrands des Hauptreflektors abwechselnd LEDs mit verschiedener Farbtemperatur angeordnet sein, wobei insbesondere insgesamt zwei unterschiedliche Farbtemperaturen vorgesehen sein können, beispielsweise 10.000 K und 2.500 K. Bei dieser Ausführungsform werden im Vergleich zu bekannten Operationsleuchten mit LEDs Farbschatten nahezu verhindert, da in diesem Fall unterschiedliche Spektralanteile unter einem annähernd gleichen Winkel in das Leuchtfeld eingestrahlt werden.

Unter LEDs bzw. Leuchtdioden im Sinne der vorliegenden Erfindung werden beliebige Leuchtdioden mit unterschiedlichen Farb- und Weißlichtanteilen und mit oder ohne eine geeignete Vorsatzoptik verstanden. Auch werden hierunter Multichip-LEDs mit unterschiedlichen Farb- und Weißlichtanteilen verstanden.

Eine besonders gute Reduzierung der Farbschatten kann dadurch erzielt werden, dass die LEDs am Außenrand des Hauptreflektors so angeordnet werden, dass sich jeweils zwei LEDs mit verschiedener Farbtemperatur diametral gegenüberliegen.

Nach einer weiteren vorteilhaften Ausführungsform der Erfindung können am Außenrand des Hauptreflektors mindesten zwei Gruppen von LEDs vorgesehen sein, wobei der Einstrahlwinkel der LEDs der ersten Gruppe und der Einstrahlwinkel der LEDs der zweiten Gruppe zur optischen Achse des Hauptreflektors unterschiedlich groß ist. Dies kann beispielsweise durch einen unterschiedlich großen radialen Abstand der LED's zur optischen Achse des Hauptreflektors oder durch eine unterschiedliche Winkelstellung der LED-Lichtquellen zur Achse des Hauptreflektors realisiert werden. In beiden Fällen ergibt sich der Vorteil, dass durch die beiden Gruppen zwei Leuchtfelder unterschiedlicher Größe erzeugt werden, die konzentrisch zueinander liegen. Hierdurch ist durch ein Dimmen der beiden LED-Gruppen durch anteiliges Überblenden der beiden Leuchtfelder eine Anpassung der Leuchtfeldgröße möglich.

Nach einer weiteren vorteilhaften Ausführungsform kann der Hauptreflektor zwischen zwei Reflektorzonen oder auch Facetten einen Abschnitt aufweisen, der so orientiert ist, dass kein Licht der LEDs reflektiert wird. Dieser Abschnitt trägt somit nicht zur Beleuchtung des Operationsfelds bei, kann jedoch dazu verwendet werden, die Höhe des Hauptreflektors zu reduzieren, indem der untere Rand einer sich distal an eine Facette anschließenden Facette in Richtung des Operationsfelds versetzt wird.

Eine besonders gute Ausleuchtung des Operationsfelds kann dadurch erreicht werden, dass als Hauptreflektor ein Freiformreflektor verwendet wird, von dem insbesondere mehrere Reflektorzonen ein und dasselbe Leuchtfeld beleuchten. Mit anderen Worten kann eine Vielzahl von Facetten, die jeweils von unterschiedlichen LEDs beleuchtet werden, auf ein und dasselbe Leuchtfeld gerichtet werden, so dass mehrere hundert Facetten dazu herangezogen werden können, ein schattenfreies Ausleuchten des Operationsfelds zu erzielen.

Schließlich kann es vorteilhaft sein, das Licht der LEDs von dem Außenrand des Hauptreflektors aus streifenförmig in diesen einzukoppeln, beispielsweise mit Hilfe einer bitorischen Linse oder einer Kombination aus Reflektor und zweistufiger Optik.

Nachfolgend wird die vorliegende Erfindung rein beispielhaft anhand vorteilhafter Ausführungsformen und unter Bezugnahme auf die beigefügten Zeichnungen beschrieben. Es zeigen:
- Fig. 1: eine schematische Querschnittsansicht einer ersten Ausführungsform einer Operationsleuchte;
- Fig. 2: eine perspektivische Ansicht von unten auf den Hauptreflektor der Ausführungsform von Fig. 1;
- Fig. 3: eine schematische Querschnittsansicht einer weiteren Ausführungsform einer Operationsleuchte ;
- Fig. 4: eine Einkopplung unter unterschiedlichen Winkeln zur Erzeugung zweier Leuchtfelder;
- Fig. 5: eine Verteilung von LEDs mit unterschiedlicher Farbtemperatur entlang des Umfangs der Operationsleuchte;
- Fig. 6: eine teilweise aufgeschnittene perspektivische Ansicht eines Optikmoduls; und
- Fig. 7: eine streifenförmige Einkopplung der LED-Strahlung mit Überlagerung einzelner Zonenanteile.

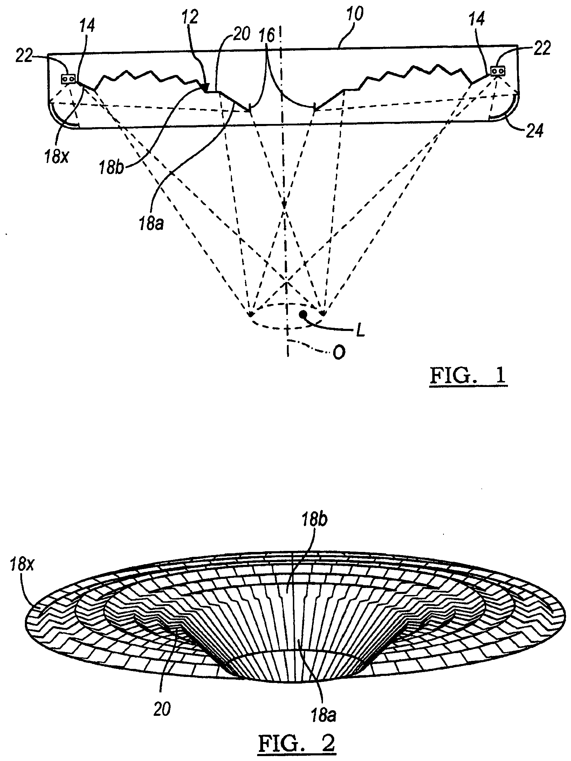

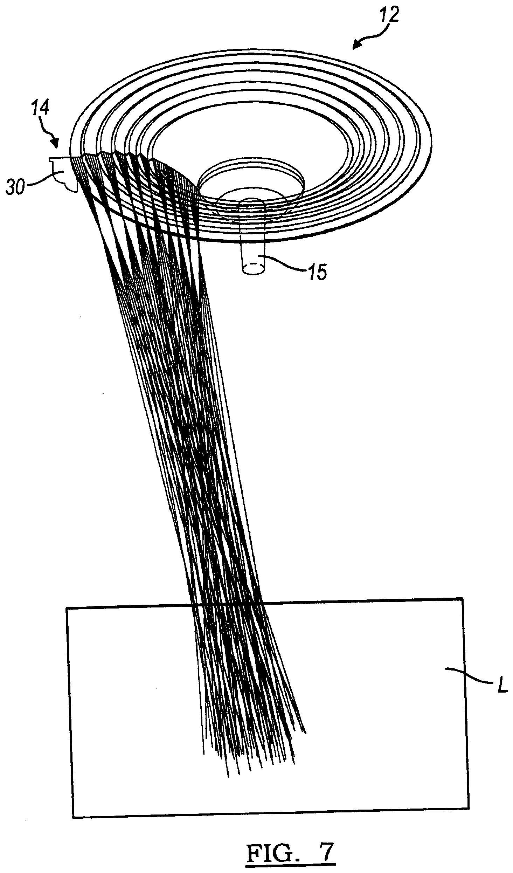

Fig. 1 zeigt eine stark schematische Querschnittsansicht einer Operationsleuchte mit einem scheibenartigen Gehäuse 10, in dem ein zentraler Hauptreflektor 12 angeordnet ist, der eine optische Achse O und einen Außenrand 14 aufweist. Bei dem dargestellten Ausführungsbeispiel ist der Hauptreflektor 12 in seinem zentralen inneren Bereich offen ausgebildet, so dass dieser Hauptreflektor 12 auch einen Innenrand 16 aufweist, der durchströmbar ist, sofern im Bereich des Gehäuses 10 entsprechende Durchströmöffnungen vorgesehen sind. Alternativ kann jedoch der Hauptreflektor 12 auch so wie in Fig. 7 dargestellt geschlossen ausgebildet sein und einen zentralen Handgriff 15 aufweisen.

Der in Fig. 1 und auch in den übrigen Figuren dargestellte Hauptreflektor 12 ist - im Gegensatz zu einem Ringreflektor oder Einzelreflektoren - flächig ausgebildet (vgl. Fig. 2) und er kann - wie bei dem dargestellten Ausführungsbeispiel - in eine Vielzahl von Reflektorzonen bzw. Facetten 18a, 18b, ..., 18x unterteilt sein, wobei der Hauptreflektor als Freiformreflektor ausgebildet ist und die einzelnen Facetten stetig oder unstetig ineinander übergehen können. Der Hauptreflektor kann beispielsweise ein beschichtetes Kunststoffspritzteil oder auch ein geprägtes und/oder gezogenes Aluminiumbauteil sein

Um die in den Figuren dargestellte äußerst flache Bauform des Hauptreflektors 12 zu erreichen, ist zwischen zwei entlang der optischen Achse benachbarten Facetten 18a und 18b ein Abschnitt 20 vorgesehen, der so orientiert ist, dass kein eingestrahltes Licht reflektiert wird. Der Abschnitt 20 trägt somit nicht zur Ausleuchtung des Leuchtfelds bei, er ermöglicht jedoch eine flache Bauform des Hauptreflektors 12, da der untere Rand, d.h. der in Richtung des Operationsfelds weisende Rand der Facette 18b entlang der optischen Achse O in Richtung des Operationsfelds versetzt ist.

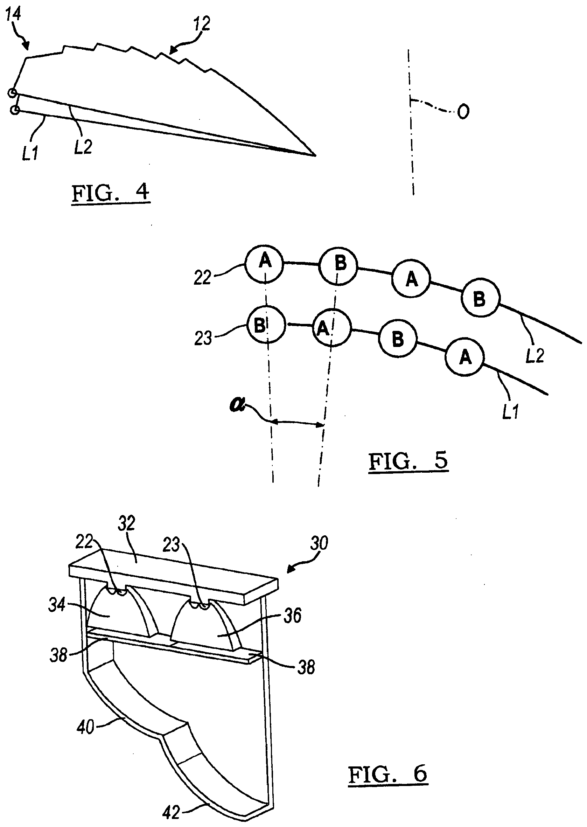

Die dargestellte Operationsleuchte weist eine Lichtquelle in Form einer Vielzahl von LEDs 22 auf, die im Bereich des Außenrands 14 des Hauptreflektors 12 ringförmig angeordnet sind, d.h. die an der Peripherie des Reflektors umlaufend angeordnet sind, und die ihr Licht von dem Außenrand 14 des Hauptreflektors aus in diesen einkoppeln. Bei dem dargestellten Ausführungsbeispiel erfolgt jedoch die Einkopplung des Lichts der LEDs 22 nicht direkt sondern indirekt über zumindest einen Einkoppelreflektor. Beim dargestellten Ausführungsbeispiel ist ein ringförmiger Einkoppelreflektor 24 verwendet, der das von den ringförmig angeordneten LEDs 22 etwa parallel zur optischen Achse O abgestrahlte Licht reflektiert und im Wesentlichen quer zur optischen Achse O lateral in den Hauptreflektor 12 einkoppelt. Hierbei ist die Geometrie des Hauptreflektors 12 so ausgelegt, dass eine Vielzahl von Facetten 18a, 18b, ..., 18x ein und dasselbe Leuchtfeld L beleuchten. Da somit eine Vielzahl von Facetten von einer Vielzahl von LEDs beleuchtet wird, ist das Leuchtfeld L auch dann nicht verschattet, wenn ein Teil des von dem Hauptreflektor 12 abgestrahlten Lichts durch den Operateur verschattet wird. Alternativ kann jedoch die Einkopplung in den Hauptreflektor auch über einzelne Einkoppelreflektoren erfolgen. Eine solche Ausführungsform ist im Zusammenhang mit Fig. 6 nachstehend detaillierter beschrieben.

Zur Reduzierung von Farbschatten können entlang des Außenrands 14 des Hauptreflektors 12 abwechselnd LEDs mit verschiedener Farbtemperatur, beispielsweise 10.000 K und 2.500 K angeordnet sein, wobei vorzugsweise insgesamt lediglich zwei unterschiedliche Farbtemperaturen abwechselnd vorgesehen sind. Hierdurch wird eine deutliche Reduzierung der Farbschatten erreicht, wobei dies noch dadurch verbessert werden kann, dass am Außenrand 14 des Hauptreflektors 12 zwei diametral gegenüberliegende LEDs 22 jeweils eine unterschiedliche Farbtemperatur aufweisen. Alternativ oder zusätzlich ist es möglich, als LEDs 22 Multichip-LEDs mit unterschiedlichen Farb- und Weißlichtanteilen inklusive einer geeigneten Vorsatzoptik zu verwenden.

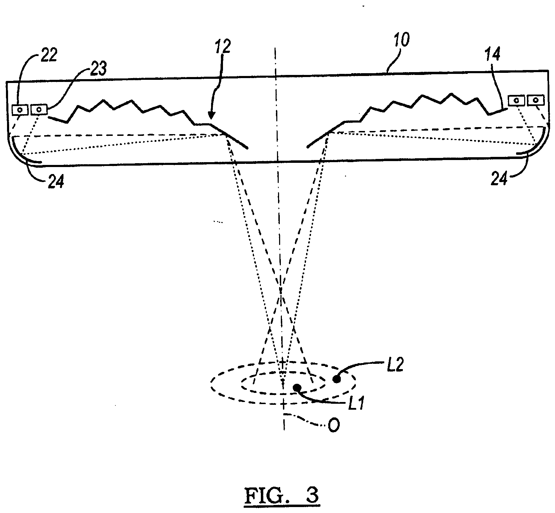

Fig. 5 zeigt eine solche abwechselnde Anordnung von LEDs mit verschiedener Farbtemperatur. Bei dem dargestellten Ausführungsbeispiel sind insgesamt nur zwei unterschiedliche Farbtemperaturen A und B, beispielsweise Kaltweiß und Warmweiß vorgesehen, wobei sich entlang des Umfangs gesehen die beiden Farbtemperaturen abwechseln. In Radialrichtung gesehen sind ebenfalls jeweils zwei unterschiedliche Farbtemperaturen hintereinander angeordnet. Der Winkelabstand α zwischen zwei benachbarten LEDs kann etwa 6° betragen. Durch den in Fig. 5 dargestellten inneren Ring von nebeneinander angeordneten LEDs wird das Leuchtfeld L1 gebildet und durch den äußeren Ring von LEDs wird das Leuchtfeld L2 gebildet. Eine Operationsleuchte mit einer solchen Anordnung von LEDs ist im Zusammenhang mit Fig.3 nachstehend näher beschrieben. Fig. 3 zeigt eine Ausführungsform einer Operationsleuchte, die im Wesentlichen derjenigen von Fig. 1 entspricht, wobei jedoch bei der in Fig. 3 dargestellten Ausführungsform eine Variation der Größe des Leuchtfelds L vorgenommen werden kann. Bei der Ausführungsform von Fig. 3 sind gleiche Bauteile mit gleichen Bezugszeichen bezeichnet und die entsprechenden Bauteile werden nicht nochmals gesondert beschrieben.

Bei der in Fig. 3 dargestellten Ausführungsform ist zusätzlich zu dem Ring von LEDs 22 ein weiterer konzentrischer Ring von LEDs 23 angeordnet, wobei die LEDs 22 in einem Abstand zur optischen Achse O angeordnet sind, der größer als der Abstand der LEDs 23 von der optischen Achse O ist. Das Licht der LEDs 22 und 23 wird jeweils auf ein und dieselben Facetten gerichtet, wobei allerdings die Einstrahlwinkel des Lichtes sowohl in den Einkoppelreflektor 24 wie auch vom Einkoppelreflektor 24 in den Hauptreflektor 12 für die beiden Gruppen von LEDs 22 und 23 unterschiedlich sind. Hierdurch kann durch ein Dimmen entweder der ersten Gruppe von LEDs 22 und/oder der zweiten Gruppe von LEDs 23 ein unterschiedlich großes Leuchtfeld L1 oder L2 erzeugt werden, so dass ohne mechanische Bewegung eine Anpassung der Leuchtfeldgröße erfolgen kann.

Das Gehäuse 10 der vorstehend beschriebenen Operationsleuchten kann entweder wie dargestellt flach und scheibenförmig ausgebildet sein. Es kann jedoch auch in seinem Zentrum durchströmbar sein. Der Hauptreflektor kann entweder die dargestellte Form oder aber die Form eines Frisbee oder einer Radkappe besitzen. Auch sind eckige Formen oder ovale Geometrien möglich.

Da die Facetten des Hauptreflektors 12 in mehrere Gruppen aufgeteilt sind, wobei jede Gruppe das eingestrahlte Licht auf eine unterschiedliche Fokussierebene abbildet, ergeben sich auch in unterschiedlichen Arbeitsentfernungen von der Operationsleuchte jeweils annähernd gleiche Lichtverhältnisse. Hierdurch kann auch der Abstand der Operationsleuchte vom Operationsfeld variiert werden, ohne dass sich die Lichtverhältnisse wesentlich ändern.

Fig. 4 verdeutlicht rein schematisch, wie durch Lichteinkopplung unter unterschiedlichen Winkeln in den Hauptreflektor 12 zwei sich überlagernde Leuchtfelder geschaffen werden können. Bei dem dargestellten Ausführungsbeispiel wird im Bereich des Außenrandes 14 des Hauptreflektors Licht unter jeweils unterschiedlichen Winkeln in den Hauptreflektor 12 eingekoppelt, wodurch die entstehenden Leuchtfelder L1 und L2 sich überlagern.

Fig. 6 zeigt als alternative Bauform zu der in Fig. 3 beschriebenen Anordnung ein Optikmodul 30, das eine LED-Platine 32 aufweist, auf der nebeneinander zwei die Strahlung vertikal nach unten richtende Leuchtdioden 22 und 23 angeordnet sind. Die Strahlung der Leuchtdioden wird dann über eine Primäroptik 34, 36, beispielsweise eine Kunststofflinse, über jeweils einen Filter 38 vertikal nach unten auf jeweils einen zugehörigen Einkoppelreflektor 40, 42 gerichtet, wobei jeder Einkoppelreflektor 40, 42 das Licht der zugehörigen LED 22 und 23 von der äußeren Peripherie des Hauptreflektors 12 aus in diesen einkoppelt.

Das in Fig. 6 dargestellte Optikmodul kann beispielsweise bei dem Ausführungsbeispiel von Fig. 3 am Außenrand 14 des Hauptreflektors 12 angeordnet werden und dort die LEDs 22, 23 sowie den Ringreflektor 24 ersetzen. Entlang des Umfangs des Hauptreflektors 12 können dann beispielsweise sechzig Optikmodule angeordnet sein.

Die Einkopplung eines einzelnen Optikmoduls, wie es in Fig. 6 dargestellt ist, ist in Fig. 7 schematisch dargestellt. Man erkennt, dass durch ein einziges Optikmodul 30 eine Einkopplung der Strahlung vom Außenumfang des Hauptreflektors 12 entlang eines radialen Streifens erfolgt, wobei die einzelnen Reflektorzonen bzw. Facetten des Hauptreflektors 12 die einfallende Strahlung nach unten auf das Lichtfeld L reflektieren, die sich dabei überlagert.

## Patentansprüche

1. Operationsleuchte, mit
einer Lichtquelle (22, 23), und
einem Hauptreflektor (12), der eine optische Achse (O) und einen Außenrand (14) aufweist und der das Licht der Lichtquelle (22, 23) auf ein Operationsfeld richtet, wobei
der Hauptreflektor (12) flächig ausgebildet ist,
der Hauptreflektor (12) in mehrere Reflektorzonen (18a, 18b, ..., 18x) unterteilt ist, die ein und dasselbe Leuchtfeld (L, L1, L2) beleuchten,
das Licht der Lichtquelle (22, 23) von dem Außenrand (14) des Hauptreflektors (12) aus in diesen im Wesentlichen quer zu seiner optischen Achse (O) eingekoppelt wird,
**dadurch gekennzeichnet, dass**
die Lichtquelle mehrere LEDs (22, 23) aufweist,
die LEDs (22, 23) am Außenrand (14) des Hauptreflektors (12) ringförmig angeordnet sind, und
die Reflektorzonen (18a, 18b, ..., 18x) in mindestens zwei Gruppen unterteilt sind, wobei jede Gruppe das einfallende Licht auf eine andere Fokussierebene abbildet.

2. Operationsleuchte nach Anspruch 1,
**dadurch gekennzeichnet, dass**
das Licht der LEDs (22, 23) über zumindest einen Einkoppelreflektor (24, 40, 42) in den Hauptreflektor (12) eingekoppelt wird.

3. Operationsleuchte nach Anspruch 2,
**dadurch gekennzeichnet, dass**
der Einkoppelreflektor ein Ringreflektor (24) ist, der insbesondere einstückig mit dem Hauptreflektor (12) ausgebildet ist.

4. Operationsleuchte nach Anspruch 2,
**dadurch gekennzeichnet, dass**
eine Vielzahl von diskreten Einkoppelreflektoren (40, 42) vorgesehen ist.

5. Operationsleuchte nach zumindest einem der vorstehenden Ansprüche,
**dadurch gekennzeichnet, dass**
entlang des Außenrands (14) des Hauptreflektors (12) abwechselnd LEDs (22, 23) mit verschiedener Farbtemperatur (A, B) angeordnet sind, wobei insbesondere insgesamt zwei unterschiedliche Farbtemperaturen vorgesehen sind.

6. Operationsleuchte nach Anspruch 5,
**dadurch gekennzeichnet, dass**
sich am Außenrand (14) des Hauptreflektors (12) zwei LEDs (22; 23) mit verschiedener Farbtemperatur (A, B) diametral gegenüberliegen.

7. Operationsleuchte nach zumindest einem der vorstehenden Ansprüche,
**dadurch gekennzeichnet, dass**
am Außenrand (14) des Hauptreflektors (12) mindestens zwei LEDs (22, 23) vorgesehen sind, deren Licht unter unterschiedlichen Winkeln in den Hauptreflektor eingekoppelt wird.

8. Operationsleuchte nach Anspruch 1,
**dadurch gekennzeichnet, dass**
der Hauptreflektor (12) zwischen zwei Reflektorzonen (18a, 18b) einen Abschnitt (20) aufweist, der so orientiert ist, dass kein Licht der LEDs reflektiert wird.

9. Operationsleuchte nach zumindest einem der vorstehenden Ansprüche,
**dadurch gekennzeichnet, dass**
zwischen dem Hauptreflektor (12) und dem Operationsfeld keine LEDs (22) angeordnet sind.

10. Operationsleuchte nach zumindest einem der vorstehenden Ansprüche,
**dadurch gekennzeichnet, dass**
der Hauptreflektor (12) ein Freiformreflektor ist.

11. Operationsleuchte nach zumindest einem der vorstehenden Ansprüche,
**dadurch gekennzeichnet, dass**
das Licht der LEDs (22, 23) von dem Außenrand (14) des Hauptreflektors (12) aus streifenförmig in diesen eingekoppelt wird, insbesondere durch eine Vielzahl von entlang der äußeren Peripherie des Hauptreflektors (12) angeordneten Optikmodulen (30).

## Claims

1. A surgical light comprising
a light source (22, 23); and
a main reflector (12) which has an optical axis (O) and an outer margin (14) and which directs the light of the light source (22, 23) onto an operating field, wherein
the main reflector (12) is formed as areal,
the main reflector (12) is divided into a plurality of reflector zones (18a, 18b, ..., 18x) which light one and the same illuminated field (L, L1, L2), and
the light of the light source (22, 23) is coupled into the main reflector (12) from its outer margin (14) and substantially transversely to its optical axis (O),
**characterized in that**
the light source has a plurality of LEDs (22, 23);
the LEDs (22, 23) are arranged in ring shape at the outer margin (14) of the main reflector (12); and
the reflector zones (18a, 18b, ..., 18x) are divided into at least two groups, with each group imaging the incident light onto a different focusing plane.

2. A surgical light in accordance with claim 1,
**characterized in that**
the light of the LEDs (22, 23) is coupled into the main reflector (12) via at least one coupling reflector (24, 40, 42).

3. A surgical light in accordance with claim 2,
**characterized in that**
the coupling reflector is a ring reflector (24) which is in particular formed in one piece with the main reflector (12).

4. A surgical light in accordance with claim 2,
**characterized in that**
a plurality of discrete coupling reflectors (40, 42) are provided.

5. A surgical light in accordance with at least one of the preceding claims,
**characterized in that**
LEDs (22, 23) with different color temperatures (A, B) are arranged alternately along the outer margin (14) of the main reflector (12), with in particular a total of two different color temperatures being provided.

6. A surgical light in accordance with claim 5,
**characterized in that**
two LEDs (22; 23) with different color temperatures (A, B) are disposed diametrically opposite one another at the outer margin (14) of the main reflector (12).

7. A surgical light in accordance with at least one of the preceding claims,
**characterized in that**
at least two LEDs (22, 23) whose light is coupled into the main reflector at different angles are provided at the outer margin (14) of the main reflector (12).

8. A surgical light in accordance with claim 1,
**characterized in that**
the main reflector (12) has a section (20) between two reflector zones (18a, 18b) which is oriented such that no light of the LEDs is reflected.

9. A surgical light in accordance with at least one of the preceding claims,
**characterized in that**
no LEDs (22) are arranged between the main reflector (12) and the operating field.

10. A surgical light in accordance with at least one of the preceding claims,
**characterized in that**
the main reflector (12) is a free-form reflector.

11. A surgical light in accordance with at least one of the preceding claims,
**characterized in that**
the light of the LEDs (22, 23) is coupled in strip form into the main reflector (12) from its outer margin (14), in particular by a plurality of optic modules (30) arranged along the outer periphery of the main reflector (12).

## Revendications

1. Lampe opératoire, comprenant
une source de lumière (22, 23) et
un réflecteur principal (12) qui présente un axe optique (O) et un bord extérieur (14) et qui dirige la lumière de la source de lumière (22, 23) sur un champ opératoire,
le réflecteur principal (12) étant de forme plate,
le réflecteur principal (12) étant divisé en plusieurs zones de réflecteur (18a, 18b, ..., 18x) qui éclairent un même champ lumineux (L, L1, L2),
la lumière de la source de lumière (22, 23) étant couplée dans le réflecteur principal (12) à partir du bord extérieur (14) de celui-ci sensiblement transversalement à son axe optique (O),
**caractérisée en ce que**
la source de lumière présente plusieurs LED (22, 23),
les LED (22, 23) sont disposées en forme d'anneau sur le bord extérieur (14) du réflecteur principal (12) et
les zones de réflecteur (18a, 18b, ..., 18x) sont divisés en au moins deux groupes, chaque groupe projetant la lumière incidente sur un plan de focalisation différent.

2. Lampe opératoire selon la revendication 1,
**caractérisée en ce que** la lumière des LED (22, 23) est couplée dans le réflecteur principal (12) par au moins un réflecteur de couplage (24, 40, 42).

3. Lampe opératoire selon la revendication 2,
**caractérisée en ce que** le réflecteur de couplage est un réflecteur annulaire (24) qui est en particulier réalisé d'une seule pièce avec le réflecteur principal (12).

4. Lampe opératoire selon la revendication 2,
**caractérisée en ce qu'**une pluralité de réflecteurs de couplage discrets (40, 42) est prévue.

5. Lampe opératoire selon au moins l'une des revendications précédentes,
**caractérisée en ce que** des LED (22, 23) ayant des températures de couleur différentes (A, B) sont disposées alternativement le long du bord extérieur (14) du réflecteur principal (12), en particulier deux températures de couleur différentes étant prévues au total.

6. Lampe opératoire selon la revendication 5,
**caractérisée en ce que** deux LED (22 ; 23) ayant des températures de couleur (A, B) différentes sont diamétralement opposées sur le bord extérieur (14) du réflecteur principal (12).

7. Lampe opératoire selon au moins l'une des revendications précédentes,
**caractérisée en ce qu'**au moins deux LED (22, 23) dont la lumière est couplée dans le réflecteur principal selon des angles différents sont prévues sur le bord extérieur (14) du réflecteur principal (12).

8. Lampe opératoire selon la revendication 1,
**caractérisée en ce que** le réflecteur principal (12) présente, entre deux zones de réflecteur (18a, 18b), une partie (20) qui est orientée de telle sorte qu'aucune lumière des LED n'est réfléchie.

9. Lampe opératoire selon au moins l'une des revendications précédentes,
**caractérisée en ce qu'**aucune LED (22) n'est disposée entre le réflecteur principal (12) et le champ opératoire.

10. Lampe opératoire selon au moins l'une des revendications précédentes,
**caractérisée en ce que** le réflecteur principal (12) est un réflecteur de forme libre.

11. Lampe opératoire selon au moins l'une des revendications précédentes,
**caractérisée en ce que** la lumière des LED (22, 23) est couplée dans le réflecteur principal (12) en bandes à partir du bord extérieur (14) de celui-ci, en particulier par une pluralité de modules optiques (30) disposés le long de la périphérie extérieure du réflecteur principal (12).
